# EUROPEAN PATENT APPLICATION

(11) **EP 0 556 106 A2**
(43) Date of publication of application: **18.08.1993**
(21) Application number: 93400308.8
(22) Date of filing: 08.02.1993
(51) Int. Cl.: C12P 21/08, G01N 33/573, C12N 5/20

(54) **Antibody binding to human aldose reductase and its uses**

(30) Priority: 12.02.1992 JP 25248/92
(71) Applicant: MITSUBISHI GAS CHEMICAL COMPANY, INC., Chiyoda-ku, Tokyo (JP)
(72) Inventor: Nishimura, Chihiro c/o C. Yabe, Yono-shi, Saitama-ken (JP); Tachikawa, Tomokazu, c/o Niigata Research Lab., Tayuhama, Niigata-shi, Niigata 950-31 (JP); Tsubouchi, Jiro, c/o Niigata Research Lab., Tayuhama, Niigata-shi, Niigata 950-31 (JP); Urakami, Teizi, c/o MITSUBISHI GAS CHEMICAL CO.INC, Tokyo 100 (JP)
(74) Representative: Bourgognon, Jean-Marie

(57) **Abstract**

Disclosed are a method of immunoassay for human aldose reductase using monoclonal or polyclonal antibodies capable of binding to human aldose reductase, and a monoclonal or polyclonal antibody and hybridoma used for the method.

## Description

The present invention relates to novel antibodies capable of binding to human aldose reductase and methods of assay for human aldose reductase using the antibodies. More particularly, the present invention relates to monoclonal anitbodies capable of binding to human aldose reductase and methods of immunoassay for human aldose reductase using the monoclonal or polyclonal antibodies. Further, the present invention relates to hybridomas producing the monoclonal antibodies.

The analysis of the function and structure of human aldose reductase has become possible with the human aldose reductase by the present invention. Also, in the field of basic medical and clinical medical sciences, it is significant to determine the content of human aldose reductase in tissues, blood, or urine with antibodies binding to human aldose reductase for understanding the dynamics of human aldose reductase.

Aldose reductase [Alditol : NAD(P)+1-oxidoreductase, EC 1.1.1.21] is an aldo-keto reductase using NAD P H as a cofactor. It has been recognized as an enzyme that regulates the development of diabetic complications such as neuropathy, nephropathy, retinopathy, and cataract (Gabbay, K. H., Merola, L. O. and Field, R. A., Science, Vol. 151, pp 209-210 (1966), Robinson, W. G. Jr., Kador, P. F. and Kinoshita, J. H., Science, Vol. 221, pp 1177-1179 (1983), Engerman, R. L and Kern, T. S., Diabetes, Vol. 33, pp 97-100 (1984), Nishimura, C., Lou, M. F., and Kinoshita, J. H., J. Neurochem., Vol. 49, pp 290-295 (1987), Kinoshita, J. H. and Nishimura, C., Diabetes Metab. Rev. Vol. 4, pp 323-337 (1988)).

Aldose reductase is participated in the metabolic process from glucose to polyol. Glucose is reduced to sorbitol by the enzyme, and sorbitol is metabolized to fructose by sorbitol dehydrogenase. An excess amount of sorbitol is produced in the cells of a tissue which uptakes glucose independent of insulin under hyperglycemia caused by diabetes. Since sorbitol has a low permeability to a cell membrane and is slowly metabolized by sorbitol dehydrogenase, it is accumulated in a large quantity in the cells of a hyperglycemic patient. A load of a high osmotic pressure on the cells with the accumulated sorbitol is considered to be a main cause for the development of diabetic complications.

Accordingly, many inhibitors to aldose reductase have been developed as prophylactic or therapeutic agents (Kador, R. F., Nakayama, T., Sato, S., Smar, M. and Miller, D. D., "Weiner, H. and Flynn, T. G., eds Progress in Clinical and Biological Research, Vol. 290, Enzymology and Molecular Biology of Carbonyl Metabolosm 2" pp 237-250 (1989), Alan, R. Liss, Inc., New York).

On the other hand, while the physiological function of aldose reductase has not yet been elucidated, there is a suggestion that the reductase is possibly participated in the regulation of the osmotic pressure in kidney medulla cells (Bagnasco, S. M., Uchida, S., Balaban, R. S., Kador, R. F. and Burg, M. B., Proc. Natl. Acad. Sci., (U.S.A.), Vol. 84, pp 1718-1720 (1987)). Also, there have been reports recently on the protein structure of aldose reductase derived from human placenta, retina and muscle (Bohren, K. M., Bullock, G., Wermuth, B. and Gabbay, K. H., J. Biol. Chem., Vol. 264, pp 9547-9551 (1989), Nishimura, C., Matsuura, Y., Kokai, Y., Akera, T., Carper, D., Morjana, N., Lyons, C. and Flynn, T. G., J. Biol. Chem. Vol. 265, pp 9788-9792 (1990)). Further, there has been a report on a genetic engineering method for preparing recombinant human aldose reductase (Nishimura et al., Biochim. Biophys. Acta., Vol. 1078, pp 171-178 (1991)).

As partly mentioned in the foregoing, in the field of basic medical and clinical medical sciences, it is very important to determine the distribution of aldose reductase in organism of a healthy, ordinary person, the amount of aldose reductase in a diabetic patient, and further, the fluctuation in the amount of aldose reductase with administration of aldose reductase inhibitors which have been already developed as therapeutic agents for diabetic complications. Thus, it has been expected to develop an antibody which is capable of binding specifically to human aldose reductase and thus can be used in immunoassay for the human aldose reductase existing in a tissue, blood, or urine and to develop a method for specifically determining the amount of human aldose reductase in a specimen with a high sensitivity and a simple procedure.

According to the present invention, there are provided antibodies capable of binding specifically to human aldose reductase and a method of immunoassay for human aldose reductase using the antibodies with a high sensitivity or a simple procedure.

Antibodies of human aldose reductase of the present invention may be either polyclonal antibodies or monoclonal antibodies.

Polyclonal antibodies can be produced by conventional methods wherein a rabbit, sheep or goat is immunized and an IgG fraction of its serum is purified.

Monoclonal antibodies can also be produced by conventional methods an example of which is briefly explained as follows:

Human aldose reductase is diluted with an isotonic sodium chloride solution to a proper concentration, made into a suspension with a complete Freund's adjuvant, and administered to a mouse or rat by intraperitoneal injection. The administration is conducted one to several times at an interval of 2 to 4 weeks with an injection of usually 10 to 100 µg of human aldose reductase. A final immunization is carried out by an intravenous injection of an isotonic sodium chloride solution containing usually 10 to 100 µg of human aldose reductase. Three to four days after the final immunization, a spleen is excised, the spleen cells and myeloma cells are fused to obtain hybridomas.

As the myeloma cell, several known cells such as a mouse SP-2, NS-1, and P3-UI, and rat Y3, Ag1, Ag2, and Ag3 may be used. In the fusional reaction of spleen cells with myeloma cells, a fusional accelerator such as a polyethylene glycol (PEG) of an average molecular weight of 1,000 to 6,000 and a Sendai virus (HVJ) can be used. The ratio of the amount of spleen cells to that of myeloma cells, both to be used, is usually about 1 to 10 spleen cells per 1 myeloma cell.

The isolation of desired hybridomas can be carried out by culturing the fused cells obtained by the method explained above in a selection medium (HAT medium) usually used for hybridoma selection and then selectively isolating growing cells. The myeloma cells mentioned above are defective in hypoxanthine-guanine phosphoribosyl transferase (HGPRT) and thus can not survive in HAT medium (medium containing hypoxanthine, aminopterin, and thymidine), while hybridoma cells can survive. Accordingly, hybridomas can be obtained by selecting growing cells.

The screening and monocloning of hybridomas which produce the objective antibodies can be conducted in accordance with a conventional limiting dilution analysis. The hybridomas which are capable of producing monoclonal antibodies of human aldose reductase can be subcultured in an ordinary culture medium and readily stored for a long period of time in a liquid nitrogen.

The hybridomas thus obtained can be grown in a neutritive culture medium or in a peritoneal cavity of a mammalia to produce antibodies. The antibodies thus produced can be purified from a supernatant of the culture medium, or the ascites or serum of the mammalia. The purification of antibodies can be effected by general isolation or purification methods such as centrifugation, dialysis, salt out with ammonium sulfate, column chromatography on DEAE-cellulose, gel filtration, affinity chromatography and the like.

The monoclonal antibodies of the present invention thus obtained have a strong capability of binding to human aldose reductase.

The monoclonal antibody of the present invention includes plural number of antibodies which will bind to different epitopes.

The human aldose reductase used for producing antibodies are not particularly restricted and may be selected from any aldose reductase derived from human being. However, the aldose reductase derived from a human placenta are preferable since they are easily available, and the aldose reductase prepared by genetic engineering are more desirable since they can be prepared in a large amount with a high purity. To be more specifically, the human aldose reductase can be obtained through genetic engineering by preparing recombinant baculoviruses containing cDNA of human aldose reductase in their genome DNA using a baculovirus expression vector, and by infecting insect cells (spodoptera frugiperda) with the recombinant virus to express human aldose reductase.

Further, it has become possible to specifically and accurately determine even a very small amount of human aldose reductase by applying enzyme immunoassay (EIA), fluorescence immunoassay (FIA), or radioimmunoassay (RIA) using the antibodies of the present invention.

EIA is known in the art, and several methods described in "Enzyme Immunoassay" (Second edition, Eiji Ishikawa et al., Igaku Shoin, (1982)) as well as in other publications may be used.

As examples of the methods, sandwich method and competition method are briefly explained as follows:

In EIA by the sandwich method, antibodies are first fixed to a solid support (first antibodies fixed to a solid support), and antigens (specimen) are added to bind to the first antibodies through antigen-antibody reaction. Then, enzyme-labelled antibodies (enzyme-labelled second antibodies) are added to bind, through antigen-antibody reaction, to the antigens which are bound to the first antibodies. Thereafter, enzyme-labelled second antibodies which were not bound to the antigens are removed, a substrate is added to effect enzyme reaction, and the amount of the antigens in the specimen is determined with a calibration curve showing the relationship between a known amount of antigens and activity of an enzymes labelled on the second antibodies.

In order to make the procedure simpler, a method (one step sandwich EIA) can been carried out wherein both antigens (specimen) and enzyme-labelled second antibodies are added simultaneously to the first antibodies fixed to a solid support.

In the sandwich method, the first antibodies and the second antibodies both of which have different recognition sites each other are usually used in combination, and the combination may be polyclonal antibodies with polyclonal antibodies, monoclonal antibodies with polyclonal antibodies, and one kind of monoclonal antibodies with another kind of monoclonal antibodies.

Each of the first antibodies and second antibodies to be used may be one kind or combination of more than one kind antibodies.

In the RIA, a method based on the same principle as that of EIA may be used.

In the competition method, antigens (specimen) and a certain amount of antibodies are allowed to react each other by antigen-antibody reaction and then the antibodies which were not bound to the antigens are allowed to react with the antigens fixed to a solid support by antigen-antibody reaction. The amount of antibodies which were bound to the antigens fixed onto the solid support is determined with enzyme-labelled antibodies.

Alternatively, antigens (specimen) and antibodies which were labelled in advance with a certain amount of enzymes (hereinafter referred to as labelled antibodies) are allowed to react through antigen-antibody reaction, the labelled antibodies which remained without binding to the antigens and the antigens fixed to a solid support are subjected to antigen-antibody reaction, and then the amount of enzymes which were bound to a solid support is determined.

The amount of enzyme can be determined by conventional methods. In any case, the amount of antigens in a specimen can be calculated with a calibration curve which was prepared using a known amount of standard antigens.

Alternatively, a mixture of certain amount of enzyme-labelled antigens and a specimen (unlabelled antigens) is contacted with antibodies fixed onto a solid support for antigen-antibody reaction so that the labelled-antigens and unlabelled-antigens compete in binding to the antibodies.

Then the amount of enzyme-labelled antigens bound to the antibodies fixed to a solid support is determined by adding a substrate of enzyme. The amount of the antigens in the specimen is calculated using a calibration curve prepared by using a known amount of standard antigens.

In both sandwich method and competition method, fragments of antibodies having a capability of binding to antigens can be used as antibody, and such fragments include fragments F(ab')₂ prepared by digestion of antibodies with pepsin, fragments Fab' obtained by reduction of the fragments F(ab')₂, and fragments Fab prepared by digestion of antibodies with papain. Also in those methods, labelled fragments prepared from the unlabelled fragments such as fragments F(ab')₂, fragments Fab', and fragments Fab can be used as labelled antibody.

An enzyme to be used for labelling antibodies or antigens include peroxidase, β-D-galactosidase, alkaline phosphatase, glucose oxidase and the like. The labelling can be effected by such methods as disclosed in "Monoclonal Antibody" (Tatsuo Iwasaki et al., Kodansha, Scientific (1984)) and "Enzyme Immunoassay" (Second edition, Eiji Ishikawa et al., Igaku Shoin, (1982)).

As for the solid support, sticks, beads, microplates, or test tubes made of silicone, nylon, plastics, or glass can be employed.

According to the present invention, immunoassay became possible by using the novel antibodies binding to human aldose reductase for determining a very small amount of human aldose reductase in a specimen, for example blood and urine.

In addition to the applications mentioned above, the antibodies of the present invention can also be used for isolation and purification of human aldose reductase from a tissue, blood or urine by a simple procedure with accurate results.

The present invention will be described in further detail with reference to Examples. However, it should be understood that the present invention is by no means restricted by such Examples.

### Example 1

### Preparation of polyclonal antibodies and enzyme-labelled polyclonal antibodies binding to human aldose reductase

(1) Preparation of the antigens used for immunization:
   In accordance with the method of Nishimura et al. (Nishimura et al., Biochim. Biophys. Acta. Vol. 1078, pp 171-178 (1991)), 10 mg of human aldose reductase was obtained.
(2) Preparation of polyclonal antibodies:
   A female rabbit of Japan White (2.0 kg) was injected subcutaneously with 500 µg of human aldose reductase with a complete Freund's adjuvant (FCA). After 3 weeks, a booster of the same amounts of human aldose reductase and FCA was used. Further, the same booster was repeated every 2 weeks, twice. Ten days after the last booster, blood was drawn from ear artery. The blood was allowed to stand for several hours at ambient temperature, left overnight at 4°C, and centrifuged for 10 minutes at 5000 x g. NaN₃ was added to the supernatant so that the final concentration of NaN₃ was 0.02 %. From the sample serum thus obtained, immunoglobrin G (IgG) fraction was isolated using a Protein A column (Ampure PA Kit, Amersham) to obtain antibodies of human aldose reductase (hereinafter referred to as the present antibody).
(3) Specificity of polyclonal antibodies:
   Western blot analysis was conducted using 1 µg/ml of the present antibody and employing both HeLa cell line derived from a human being, and extracts of a human tissue as test objects. As the result, a single band of protein which reacts with the antibodies was observed at the same position as the human aldose reductase.
   Further, a cross-reaction of the present antibody with aldehyde reductase which exist in a large amount in a human tissue as a related enzyme of aldose reductase was investigated as follows:
   First, a mixture of 0.1 µg of aldose reductase and 0.1 µg of human renal aldehyde reductase and a mixture of 0.1 µg of aldose reductase and 0.2 µg of human renal aldehyde reductase were respectively subjected to SDS-polyacrylamide gel electrophoresis and then Western blot analysis was conducted using the antibodies. As the result, the human aldose reductase was detected while the human renal aldehyde reductase was not detected.
(4) Preparation of enzyme-labelled polyclonal antibodies:
   Polyclonal anitbodies of human aldose reductase were dialysed against a phosphate buffered saline (5mM sodium phosphate, 150 mM NaCl, (pH 7.5)), diluted to give a concentration of 1 mg/ml, and then labelled with alkaline phosphatase according to the instructions of Immuno-Link AP Labelling Kit (Cambridge Research Biochemicals, Cheshire CW9 7RA). Enzyme-labelled polyclonal antibodies in an amount of 4.5 µg/ml were used for EIA of human aldose reductase.

### Example 2

### Measurement of human aldose reductase using polyclonal antibodies binding to human aldose reductase

A procedure of enzyme immunoassay (EIA, sandwich method) is explained as follows:
(1) First, 5 µg/ml of the polyclonal antibodies in Example 1 diluted with 50 µl of 50 mM sodium carbonate buffer (pH 9.6) is added into each of the wells of two or more immuno microplates (Nunc-Immuno Plate, MaxiSorp) of 96 wells. The wells were sealed, and the solution in the wells was lightly stirred with a mixer, and allowed to stand for more than 5 hours at 4°C to fix the antibodies on a solid support. After removing the solution of the present antibody, the plate was washed 3 times with deionized water.
(2) Next, 100 µl of a blocking solution (170 mM H₃BO₃, 120 mM NaCl, 0.05 % Tween 20, 1 mM EDTA, 0.25 % bovin serum albumin, 0.05 % NaN₃, (pH 8.5)) was added into each of the wells. The wells were sealed. The solution was stirred with a mixer and allowed to stand for 30 minutes at ambient temperature. After removing the blocking solution, the plate was washed 3 times with deionized water.
(3) A specimen was diluted with 4.5 µg/ml of a solution of enzyme-labelled polyclonal antibodies, and 50 µl of its portion was added to each of some wells. The wells were sealed, the plate was fixed to a plate mixer, and incubated at 4°C overnight with gentle agitation. Unaltered polyclonal antibodies were removed and then the plate was washed 3 times with deionized water. In this procedure, the specimen was serially diluted in duplicate. Further, 5 to 50 ng/ml of a standard antigen solution was added into each of remaining wells to construct a standard curve.
(4) Next, 100 µl of a blocking solution (composition was the same as in (2) above) was added into each of the wells. The wells were sealed. The solution was lightly stirred with a mixer, allowed to stand for 10 minutes at ambient temperature. The blocking solution was removed and then the plate was washed 3 times with deionized water.
(5) Then, 50 µl of diethanolamine buffer solution (10 mM diethanolamine-HCl, 0.5 mM MgCl₂, (pH 9.8)) containing 1 mg/ml of p-nitrophenyl phosphate substrate and 1 mM of lavamisole was added to each of the wells, and sealed. The solution was lightly stirred with a mixer and allowed to stand for 1 to 3 hours (until the absorbance of the wells added with 50 ng/ml of the present antigen reached about 1.0-1.5) at a temperature of 27°C.

Absorbance by each of the wells was determined at 405 nm with an Immuno Plate Reader, and the amount of the present antigen in each specimens was calculated using the standard curve prepared from the measurements of a diluted solution of the present antigen.

Human aldose reductase and human renal aldehyde reductase were diluted to 5-50 ng/ml, respectively, according to the enzyme immunoassay explained above, and the reaction was conducted on the same plate. The results are shown in Table 1.

**Table 1**

| Measurement of aldose reductase and aldehyde reductase by enzyme immunoassay (Standard curve) | |
|---|---|
| Specimen | Absorbance at 405 nm |
| Aldose reductase | |
| 0 (ng/ml) | 0.080 |
| 5 | 0.200 |
| 10 | 0.360 |
| 15 | 0.470 |
| 22 | 0.620 |
| 30 | 0.820 |
| 40 | 0.900 |
| 50 | 0.970 |

| Aldehyde reductase | |
|---|---|
| 0 (ng/ml) | 0.080 |
| 5 | 0.080 |
| 10 | 0.090 |
| 15 | 0.090 |
| 22 | 0.090 |
| 30 | 0.100 |
| 40 | 0.100 |
| 50 | 0.110 |

The absorbance by the human aldose reductase increased with increasing concentration of the antigens, while the increase of the absorbance by the human aldehyde reductase was not observed in this assay. This result indicates the specificity of the present assay to the human aldose reductase, and it can be understood that it is preferable for the determination of human aldose reductase.

### Example 3

### Measurement of aldose reductase in human red blood cells using polyclonal antibodies binding to human aldose reductase

To 0.5 to 1.0 ml portion of heparinized whole blood drawn from veins of healthy 7 men and 5 women aged 21 to 37 was added the same volume of an ACD solution (acid-citrate-dextrose solution; 23 mM citric acid, 44.9 mM sodium citrate, 81.7 mM dextrose), and kept at 4°C to use as specimen. Then 10 ml of an ice-cold phosphate buffer saline (composition was the same as in Example 1, (4) above) was added to each of the specimens, mixed, and centrifuged at 4°C for 10 minutes at 1,500 x g. The supernatant was removed, 10 ml of the ice-cold phosphate buffer saline was further added, and centrifuged under the same conditions. This procedure was repeated twice. The packed red blood cells were stored frozen at -80°C.

Then, frozen red blood cells were thawed and subjected twice to the re-freezing procedure with dry ice-acetone to effect complete hemolysis of the red blood cells.

Thereafter, centrifugation was conducted to obtain the hemolysate, and the amount of aldose reductase contained in the hemolysate was determined by the enzyme immunoassay in Example 2. The amount of hemoglobin contained in the hemolysate was also determined by cyanmethohemoglobin method (Hemoglobin-testwako, Wako Junyaku). The results are shown in Table 2.

**Table 2**

| Specimen | A: Aldose reductase (ng/ml hemolysate) | B: Hemoglobin (ng/ml hemolysate) | A/B |
|---|---|---|---|
| Man 1 | 815 | 248 | 3.28 |
| 2 | 1,810 | 227 | 7.97 |
| 3 | 784 | 246 | 3.19 |
| 4 | 773 | 194 | 3.99 |
| 5 | 910 | 225 | 4.04 |
| 6 | 1,066 | 197 | 5.41 |
| 7 | 1,269 | 184 | 6.90 |
| Woman 1 | 953 | 254 | 3.75 |
| 2 | 800 | 225 | 3.55 |
| 3 | 1,067 | 233 | 4.58 |
| 4 | 1,342 | 200 | 6.71 |
| 5 | 759 | 239 | 3.18 |

As will be understood from Table 2, the amount of aldose reductase varied over a wide range of from about 750 ng/ml hemolysate to about 1,800 ng/ml hemolysate depending on individuals, and the relative value to hemoglobin varied from about 3 to about 8.

### Example 4

### Preparation of monoclonal antibodies binding to human aldose reductase

(1) Preparation of antigens used for immunization:
   Preparation of the antigens was carried by the same method as in Example 1.
(2) Preparation of immunosplenocytes:
   An emulsion of 50 µg of human aldose reductase and a complete Freund's adjuvant was administered in an intraperitoneal cavity of a male Balb/c mouse of 6 to 8 weeks age. Three weeks after, an isotonic sodium chloride solution containing 50 µg of human aldose reductase was administered in a vein of the mouse. Three days after the final immunization, the mouse was sacrificed, and its spleen was exercised to use for cell fusion.
(3) Preparation of hybridomas:
   Cells obtained by spreading the spleen to pieces with tweezers were suspended in a culture medium which was prepared by adding 0.29 g/l of L-glutamine, 0.11 g/l of pyravic acid, 0.06 g/l of kanamycin sulfate, 0.06 g/l of crystalline penicillin G·K, and 1.2 g/l of NaHCO₃ to a culture medium, RPMI-1640 medium ("Nissui" 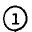 made by Nissui Seiyaku, hereinafter referred to as RPMI-1640 culture medium), and passed through a nylon mesh to obtain a suspension of single cells. The single cell suspension was treated with a mixture of 0.83 % ammonium chloride solution (9 parts by volume) and 0.17 M of tris(hydroxymethyl)aminomethane hydrochloride buffer solution (pH 7.6) (1 part by volume) for 2 minutes at 4°C to destroy red blood cells in the single cell suspension, and then the red blood cells were removed by centrifugation.
   Mouse myeloma cells SP-2 at a logarithmic growth phase which were cultured in RPMI-1640 culture medium added with 10 % bovine fetal serum (hereinafter referred to as 10 % FCS RPMI-1640 culture medium) was washed twice with RPMI-1640 culture medium. Immunosplenocytes and the mouse myeloma cells SP-2 were suspended in the culture medium RPMI-1640 at a ratio of 5:1 and homogenized. Then, the cells were recovered by centrifugation and heated to 37°C in an aqueous solution.
   Next, 2.0 ml of 50 % polyethylene glycol solution (produced by Boeringer Manheim Yamanouchi Seiyaku) of an average molecular weight of 1,500 and heated to 37°C in advance was gradually added to the cell suspension in 1 minute, and then cell fusion reaction was further conducted for 1 minute. The RPMI-1640 culture medium in an amount of 10 ml was added dropwise into the mixture over 4 minutes to terminate the cell fusion reaction, and then the whole suspension was centrifuged to obtain cells. The cells were suspended in a 10 % FCS-RPMI-1640 culture medium to give a cell concentration of 5 x 10⁶ cells/ml, then 100 µl portions of the suspension was added to each of the wells of a microplate of 96 wells (produced by Corning Glass Work). The cells in the wells of the microplate were cultured for 1 day in an atmosphere containing 5.5 % of carbon dioxide gas at 37°C, and then 100 µl of a 10 % FCS-RPMI-1640 culture medium containing 4 x 10⁻⁷ M of aminopterin, 1.6 x 10⁻⁵ M of thymidine, and 1 x 10⁻⁴ M of hypoxanthine (hereinafter referred to as HAT culture medium) was added to each of the wells. One day after, a half of the culture medium was removed by aspiration from each of the wells, then 100 µl of the HAT culture medium was added to each of the wells. Thereafter, every 2 or 3 days, half of the culture medium was exchanged for a new culture medium and the culture was continued. Ten days after the cell fusion growth of hybridomas was observed.
(4) Selection of hybridomas producing monoclonal antibodies binding to human aldose reductase:
   The screening of the antibodies capable of binding to human aldose reductase in the supernatant of hybridoma culture was carried out with EIA using a microplate coated with human aldose reductase. The fixation of the human aldose reductase was carried out as follows:
   To each of the wells of an immunomicro plate (produced by Nunc-Immuno Plate, Maxisorp) having 96 wells was added 100 µl of human aldose reductase solution prepared by 0.01 M phosphate buffer saline containing 0.15 M of NaCl (pH 7.4) (hereinafter referred to as PBS) so that the concentration of the reductase was 0.5 µg/ml. Then the plate was allowed to adsorption for 2 hours at ambient temperature.
   The microplate was washed 5 times with PBS. A PBS solution containing 0.2 % of bovine serum albmin and 0.05 % of Tween 20 (hereinafter referred to as BSA-Tween 20-PBS) was added to each of the wells, and the wells were completely blocked to avoid non-specific absorption of a protein.
   A supernatant of the hybridoma culture obtained by the procedure described in (3) above was diluted with PBS, and 100 µl of the diluted solution was added to each of the wells and allowed to react for 1 hour at an ambient temperature to bind monoclonal antibodies to the enzymes fixed to the wells. Detection of the monoclonal antibodies bound to the enzymes was carried out by using a VECTASTAIN ABS Kit (produced by Vector Laboratories). Namely, the plate was washed 5 times with a PBS containing 0.05 % of Tween 20 (hereinafter referred to as Tween 20-PBS solution), added with 100 µl of biotinized goat antimouse immunoglobin antisera (BSA-Tween 20-PBS solution), allowed to react for 1 hour, washed with Tween 20-PBS, added with 100 µl of avidinized peroxidase (BSA-Tween 20-PBS solution), and then allowed to react for 20 minutes. The plate was washed 5 times with Tween 20-PBS. Then, 100 µl of a mixed solution of the same volume of 0.03 % H₂O₂ and 100 mM citric acid buffer solution (pH 5.0) containing 1 mg/ml of o-phenylene diamine dihydrochloride was added to each of the wells, and subjected to incubation for 10 minutes at ambient temperature.
   Thereafter, 100 µl of 2N sulfuric acid was added to each of the wells to terminate the reaction, and absorbance of the solution of the reaction product was determined using an Immuno Reader NJ-2000 (produced by Inter Med Corporation).
   As the result, of total 712 wells added with the fused cells, growth of hybridomas was observed with 661 wells, among which 89 were positive wells producing an antibodies capable of binding to human aldose reductase. With 40 wells having a particularly strong antibody activity, cloning of hybridomas was carried out.
(5) Cloning of hybridomas producing monoclonal antibodies binding to human aldose reductase:
   A culture medium of hybridomas having an antibody activity and capable of binding to human aldose reductase was transferred to a flat bottom microplate of 24 wells (produced by Corning Glass Works) having thymocytes of a Balb/c mouse as a feeder layer (1 x 10⁷ cell/ml). Grown hybridomas were cloned with a microplate of 96 wells having thymocytes of a Balb/c mouse as a feeder layer (1 x 10⁷ cell/ml) by the limiting dilution method. Cloning operation was repeated twice to obtain 18 clones in total.
(6) Purification of monoclonal antibodies binding to human aldose reductase:
   The hybridoma cells obtained by the procedure described in (5) above which produce monoclonal antibodies capable of binding to human aldose reductase were transplanted in an amount of 2-5 x 10⁶ in an intraperitoneal cavity of a Balb/c mouse which was given 0.5 ml of Pristane 10 and 3 days before the transplantation, respectively. About 1 week after, ascites of the mouse was harvested, and crude monoclonal antibodies were obtained from the ascites by salting out with 50 % saturated ammonium sulfate solution. The monoclonal antibodies were dissolved in a small amount of PBS, and purified with an Affi-Gel Protein A-MAPS Kit (Bio-Rad Laboratories). Then, the solution of the antibodies was dialysed against PBS, and stored at 4°C. Purified antibodies were obtained in a yield of 0.5 to 10 mg per 1 ml of ascites.

### Example 5

### Characteristics of monoclonal antibodies binding to human aldose reductase

(1) Determination of immunoglobulin subclass of monoclonal antibodies:
The immunoglobulin subclass of the purified antibodies were determined by Ouchterlony test with class-specific goat mouse immunoglobulin antisera (Meroy Laboratories). The results are shown in Table 3.

**Table 3**

| Antibody | Immunoglobulin subclass |
|---|---|
| ARmAb 2 | IgG₁ |
| ARmAb 3 | IgM |
| ARmAb 5 | IgG₁ |
| ARmAb 7 | IgG₁ |
| ARmAb 9 | IgG₁ |
| ARmAb11 | IgG₁ |
| ARmAb19 | IgG₁ |
| ARmAb20 | IgG₁ |
| ARmAb21 | IgG₁ |
| ARmAb22 | IgG₁ |
| ARmAb23 | IgG₁ |
| ARmAb25 | IgG₁ |
| ARmAb26 | IgM |
| ARmAb29 | IgG₁ |
| ARmAb30 | IgG₁ |
| ARmAb35 | IgG₁ |
| ARmAb36 | IgG₁ |
| ARmAb37 | IgG₁ |

All of the monclonal antibodies except ARmAb3 and ARmAb26 were IgG₁.
(2) Reactivity with human aldose reductase and human aldehyde reductase:
The reactivity of monoclonal antibodies (capable of binding to human aldose reductase) with human aldose reductase and the reactivity of the same monclonal antibodies with human aldehyde reductase were investigated by EIA using a microplate coated with human aldose reductase or human aldehyde reductase. The fixation of the human aldose reductase or human aldehyde reductase to the microplate was carried out by the same method as in Example 4, (4). After the fixation, 100 µl of a solution of monoclonal antibodies (0.1 µg/ml to 100 µg/ml) dissolved in BSA-Tween 20-PBS was added to the wells of the plate, and incubated for 1 hour at ambient temperature.
Detection of antibodies bound to the microplate having human aldose reductase or human aldehyde reductase fixed thereto was carried out by the same method as in Example 4, (4) using a VECTASTAIN ABS Kit. The results of addition of 0.1 µg/ml of monoclonal antibodies to the microplate coated with human aldose reductase and the results of addition of 100 µg/ml of monoclonal antibodies to the microplate coated with human aldehyde reductase are shown in Table 4.

**Table 4**

| Absorbance at 490 nm | | |
|---|---|---|
| ARmAb | Aldose reductase (AR) | Aldehyde reductase (ALR) |
| 2 | 2.63 | 0.00 |
| 36 | 0.67 | 0.00 |
| 25 | 1.99 | 0.00 |
| 21 | 2.15 | 0.00 |
| 37 | 1.67 | 0.41 |
| 19 | 1.01 | 0.37 |
| 35 | 0.83 | 0.31 |
| 7 | 0.48 | 0.71 |

As the results, ARmAb2, ARmAb36, ARmAb25, and ARmAb21 were found to be the antibodies that bind to human aldose reductase but do not bind to human aldehyde reductase. Thus, they are preferable for measurement specific to AR. On the other hand, ARmAb37, ARmAb19, ARmAb35, and ARmAb7 were found to be the antibodies that slightly bind to human aldehyde reductase.
(3) Difference of human aldose reductase epitopes (sites) to which monoclonal antibodies bind:
In order to determine whether human aldose reductase epitopes (sites) to which monoclonal antibodies bind are the same or different, each of the antibodies was labelled with horseradish peroxidase (hereinafter referred to as HRP) and subjected to competition reaction with each of unlabelled antibodies toward human aldose reductase. The difference was investigated through the observation of existence of the inhibition. The labelling of the antibodies with HRP was carried out by periodic acid oxidation method ("Monoclonal Antibody", Tatsuo Iwasaki et al., Kodansha, Scientific, (1984)).

### (Labelled antibodies)

To a solution containing 4 mg of HRP (produced by Toyobo) in 1 ml of distilled water was added 200 µl of 0.1 M sodium periodate, and then allowed to react for 20 minutes at ambient temperature. The solution thus obtained was dialysed overnight at 4°C against 1 mM sodium acetate buffer solution (pH 4.4). To the dialysate (inner solution after the dialysis) was added 20 µl of 0.2 M sodium carbonate to adjust the pH of the solution to 9.0. Immediately after the pH adjustment, a solution of antibodies prepared by dissolving each antibodies in 0.01 M sodium carbonate buffer solution (pH 9.5) to give a concentration of 5 mg/ml was added to the solution, stirred for 2 hours at ambient temperature, cooled on ice, added with 0.1 ml of NaBH₄, and allowed to react for 2 hours at 4°C.

The solution of the reaction product was dialysed against PBS at 4°C overnight and subjected to gel filtration with a Superose 12 column (produced by Pharmacia Fine Chemicals) equilibrated with PBS to obtain a fraction having absorbance both at 280 nm and 403 nm. That was a fraction of the antibodies labelled with HRP. Unreacted HRP and antibodies were separated. All the antibodies thus obtained were found to be labelled and retaining the binding activity to human aldose reductase.

### (Competition Reaction)

First, 100 µl of a PBS solution (0.2 µg/ml) of a human aldose reductase was added to each of the wells of the immuno microplate, and allowed for adsorption. The plate was washed 3 times with PBS, and BSA-PBS was added to each of the wells to completely block the wells to avoid any non-specific adsorption of proteins to the wells.

After 100 µl of solution containing antibodies labelled with HPR and 100 µl of unlabelled antibodies solution (0 to 1 mg/ml) diluted serially with Tween 20-PBS containing 0.2 % of BSA (hereinafter referred to as BSA-Tween 20-PBS) were added simultaneously to each of the wells and subjected to antigen-antibody reaction for 1 hour at ambient temperature, the plate was washed 4 times with Tween 20-PBS.

Then, 100 µl of a substrate containing o-phenylene diamine (solution prepared by dissolving H₂O₂ and o-phenylene diamine dihydrochloride in 100 mM citric acid buffer solution (pH 5.0) so that their concentration were 0.015 % and 1 mg/ml, respectively, hereinafter the same definition will be applied) was added to each of the wells, allowed to react for 30 minutes at ambient temperature. Thereafter, the wells were added with 100 µl of 2N sulfuric acid to terminate the reaction.

The absorbance by the solution of the reaction product was determined at 490 nm using an Immuno Reader NJ-2000. The results are shown in Table 5.

**Table 5**

| Antibody | Enzyme-labelled antibody (HRP) | | | | | |
|---|---|---|---|---|---|---|
| | ARmAb 2 | ARmAb 7 | ARmAb19 | ARmAb21 | ARmAb25 | ARmAb35 |
| ARmAb 2 | + | - | - | - | - | - |
| ARmAb36 | - | - | - | - | - | - |
| ARmAb25 | - | + | + | + | + | + |
| ARmAb21 | - | + | + | + | + | + |
| ARmAb37 | - | + | + | + | + | + |
| ARmAb19 | - | + | + | + | + | + |
| ARmAb35 | - | + | + | + | + | + |
| ARmAb 7 | - | + | + | + | + | + |

As shown in Table 5, ARmAb 2 and ARmAb36 did not compete with any other antibodies, but other 6 monoclonal antibodies competed with each other. From the results, it has been found out that ARmAb 7, ARmAb19, ArmAb21, ARmAb25, ARmAb35, and ARmAb37 are the antibodies that bind to all the same or very close sites of human aldose reductase, while ARmAb 2 and ARmAb36 are the antibodies that bind to the sites different from those 6 monoclonal antibodies.

### Example 6

### Measurement of human aldose reductase using monoclonal antibodies binding to human aldose reductase (EIA by sandwich method)

When monoclonal antibodies are used in the sandwich method, correct measurement is practically impossible if the same antibodies or the antibodies having similar binding sites were used in combination both for the first antibodies (antibodies fixed onto a solid support) and for the second antibodies (enzyme-labelled antibodies). However, from the results of the experiments showing the inhibition to competition reaction in Example 5, (3), usable combinations of the antibodies can be determined.

In the following, there will be illustrated an example in which ARmAb21 and HRP-labelled ARmAb36 were used as the first and second antibodies, respectively, and another example in which ARmAb36 and HRP-labelled ARmAb25 fragments Fab' were used as the first and second antibodies, respectively. In addition to these combinations, measurements of human aldose reductase were also possible with other combinations of the antibodies.

### (a) Two step sandwich EIA:

A solution of ARmAb21 was prepared with PBS to give a concentration of 5 µg/ml, and 100 µl of its portion was added to each of the wells of an immuno microplate, and left for 2 hours at ambient temperature for adsorption. The plate was washed 3 times with PBS and subjected to blocking with BSA-PBS for 1 hour at ambient temperature to prevent non-specific adsorption.

Then, 100 µl of human aldose reductase solution diluted with BSA-Tween 20-PBS were added to the wells and allowed to react for 2 hours at ambient temperature. The plate was washed 3 times with Tween 20-PBS. Thereafter, ARmAb36 labelled with HRP was diluted with BSA-Tween 20-PBS to give a concentration of 1 µg/ml, 100 µl of its portion was added to each of the wells and allowed to react for 2 hours at ambient temperature, and the plate was washed 3 times with BSA-Tween 20-PBS, as in Example 5, (3).

To the wells was added 100 µl of a substrate containing o-phenylendiamine dihydrochloride and allowed to react for 30 minutes at ambient temperature. Then, 100 µl of 2N sulfuric acid was added to each of the wells to terminate the reaction, and the absorbance of the reaction product was measured at 490 nm with an Immuno Reader NJ-2000. The results are shown in Table 6.

**Table 6**

| Aldose reductase (ng/ml) | Absorbance at 490 nm |
|---|---|
| 0 | 0.004 |
| 1 | 0.065 |
| 5 | 0.355 |
| 10 | 0.750 |
| 15 | 1.148 |
| 20 | 1.528 |

As will be understood from Table 6, the concentration of human aldose reductase and the absorbance had a rectilinear relationship.

### (b) One step sandwich EIA:

An example of one step sandwich method for immuno assay for human aldose reductase is given in the following wherein ARmAb36 was used as the first antibody to be fixed onto a solid support and HRP-labelled fragments Fab' of ARmAb25 prepared through digestion with pepsin (hereinafter referred to as HRP-labelled Fab') was used as the second antibody.

### (Preparation of HRP-labelled Fab')

To 10 ml of a PBS solution containing 2 mg/ml of ARmAb25, 1 M citric acid buffer solution (pH 3.2) was added to adjust the pH of the PBS solution to 4.0. To this solution was added 250 µg of pepsin derived from a porcine stomach mucosa (produced by Sigma Chemical Company), and incubated for 12 hours at 37°C to digest the antibodies. The pH of the solution was adjusted to 7.0 by the addition of 3 M tris-hydrochloric acid buffer solution (pH 8.6) and concentrated to 2 ml. The concentrated solution was applied to a Superose 12 HR 16/50 column (produced by Pharmacia Fine Chemicals) equilibrated with 0.1 M phosphate buffer solution (pH 6.0) to afford F(ab')₂ fraction. Thus, 4.5 mg of F(ab')₂ was recovered.

The F(ab')₂ fraction was concentrated to 2 ml, and 2-mercaptoethylamine and EDTA were added to the concentrated solution to give a final concentration of 10 mM and 0.5 mM, respectively. The mixture was then incubated for 90 minutes at 37°C to reduce F(ab')₂.

The solution thus obtained was applied to a Superose 12 HR 16/50 column equilibrated with 0.1 M phosphate buffer solution (pH 6.0) containing 5 mM of EDTA to afford a Fab' fraction. Thus, 3.8 mg of Fab' was recovered.

The introduction of maleimide groups into the HRP was carried out by dissolving 10 mg of HRP in 0.1 M phosphate buffer solution (pH 7.0), adding 150 µl portion of a solution prepared by dissolving 10 mg of N-(ε-maleimidecaproyloxy)succinimide in N,N-dimethyl formamide, and allowing to react for 60 minutes at 30°C.

The solution of the reaction product was applied to a PD 10 column (produced by Pharmacia Fine Chemicals) equilibrated with 0.1 M phosphate buffer solution (pH 6.0) to afford a HRP fraction having maleimide groups introduced therein.

Then, 0.8 ml of the maleimide groups-introduced HRP having a concentration of 3.6 mg/ml was added to 2 ml of 1.5 mg/ml Fab' solution prepared by the procedure mentioned above, and allowed to react for 60 minutes at 30°C. The solution of the reaction product was concentrated to 1.5 ml and applied to a Superose 12 HR 16/50 column equilibrated with 0.1 M phosphate buffer solution (pH 6.5) to afford 10 ml of HRP-labelled fragments Fab'. The molar ratio of HRP to Fab' was 1:1 in the HRP-labelled Fab' thus prepared.

ARmAb36 was prepared into a solution having a concentration of 10 µg/ml with 50 mM carbonate buffer solution (pH 9.5), a 200 µl of its portion was added to each of the wells of an immuno microplate, and allowed to adsorption for 2 hours at ambient temperature. The immuno microplate was washed 3 times with PBS, each of its wells was filled with BSA-PBS, and then subjected to blocking for 1 hour at ambient temperature to prepare a plate fixed with antibodies.

The HRP-labelled Fab' prepared as above was diluted to 200 ng/ml with BSA-Tween 20-PBS and 100 µl of its portion was added to each of the wells. Then, 100 µl of a solution of human aldose reductase diluted serially with BSA-Tween 20-PBS was further added to each of the wells, allowed to stand for 2 hours at ambient temperature, and then washed 3 times with Tween 20-PBS. Thereafter, 200 µl of a substrate solution containing o-phenylene diamine dihydrochloride was added to each of the wells, allowed to react for 30 minutes at ambient temperature. To this plate was added 100 µl of 4N sulfuric acid to terminate the reaction, and absorbance by the solution of the reaction product was determined at 490 nm with an Immuno Reader NJ-2000.

The relationship between the concentration of human aldose reductase and the absorbance is shown in Table 7.

**Table 7**

| Aldose reductase (ng/ml) | Absorbance at 490 nm |
|---|---|
| 0 | 0.021 |
| 5 | 0.265 |
| 10 | 0.531 |
| 15 | 0.792 |
| 20 | 1.033 |

As shown in Table 7, the concentration of aldose reductase had a rectilinear relationship with absorbance.

### Example 7

### Measurement of human aldose reductase using monoclonal antibodies and polyclonal antibodies binding to human aldose reductase ( EIA sandwich method using monoclonal antibodies and polyclonal antibodies)

To each of the wells of an immuno microplate of 96 wells (Nunc-Immuno-Plate, Maxisorp) was added 50 µl of 5 µg/ml solution of monclonal antibodies, ARmAb25 diluted with 50 mM sodium hydrogencarbonate buffer solution (pH 9.6).

The plate was sealed, the solution in the wells was lightly stirred with a mixer, and the plate was allowed to stand for more than 5 hours at 4°C to fix the antibodies onto a solid support.

After removing the solution of the antibodies, the plate was washed 3 times with deionized water. Then, 100 µl of a blocking solution (170 mM H₃BO₃, 120 mM NaCl, 0.05 % Tween 20, 1 mM EDTA, 0.25 % bovine serum albumin, 0.05 % NaN₃ (pH 8.5)) was added to each of the wells. The wells were sealed. The solution was stirred with a mixer and allowed to stand for 30 minutes at ambient temperature. Thereafter, the blocking solution was removed and the plate was washed 3 times with deionized water.

Next, 50 µl of human aldose reductase solution diluted with the blocking solution mentioned above was added to each of the wells and allowed to react for 2 hours at ambient temperature. The plate was washed 3 times with deionized water, 100 µl of a blocking solution (composition was the same as above) was added to each of the wells. The wells were sealed. The solution was lightly stirred, and allowed to stand for 10 minutes at ambient temperature. The blocking solution was removed and the plate was washed 3 times with deionized water.

Then, 50 µl of a 4.5 µg/ml solution of the enzyme-labelled polyclonal antibodies prepared in Example 1 was added to each of the wells, allowed to react for 2 hours at ambient temperature. The plate was washed 3 times with deionized water, and 100 µl of the blocking solution was further added to each of the wells, stirred, and allowed to stand for 10 minutes at ambient temperature. The plate was washed 3 times with deionized water.

Finally, each 75 µl of p-nitrophenyl phosphate solution (1 mg/ml) dissolved in diethanolamine buffer solution (10 mM diethanolamine-HCl, 0.5 mM MgCl₂, (pH 9.8)) and levamisole (1 mM) was added to each of the wells, sealed, lightly stirred with a mixer, allowed to stand for 1 to 3 hours (until absorbance of the wells added with 50 ng/ml of the present antigens reached about 1.0) at 27°C, and subjected to determination of absorbance at 405 nm with a plate reader. The results are shown in Table 8.

**Table 8**

| Aldose reductase (ng/ml) | Absorbance at 490 nm |
|---|---|
| 0 | 0.030 |
| 1 | 0.030 |
| 5 | 0.060 |
| 15 | 0.167 |
| 30 | 0.373 |
| 50 | 0.690 |

The concentration of the human aldose reductase had a rectilinear relationship with absorbance.

### Example 8

### Measurement of aldose reductase in red blood cells of 10 diabetic patients

An ACD solution (composition was the same as in Example 3) was added to 0.5 to 1.0 ml of heparinized whole blood drawn from the veins of diabetic patients and stored at 4°C to use as specimen. To each of the specimens was added 10 ml of an ice-cold phosphate buffered saline (composition was the same as above).

The solution was mixed, centrifuged at 4°C for 10 minutes at 1,500 x g. The supernatant was removed. Further, the operation of the addition of 10 ml of ice-cold phosphate buffered saline and centrifugation under the same conditions were repeated twice, and the packed red blood cells were stored frozen at -80°C.

Then, frozen blood cells were thawed, and subjected twice to the re-freezing procedure with dry ice-acetone to effect complete hemolysis of the red blood cells.

Thereafter, centrifugation was conducted to obtain the hemolysate. The amount of aldose reductase contained in the hemolysate was determined by the sandwich EIA in Example 2 using polyclonal antibodies-polyclonal antibodies (poly-poly method) and by the sandwich EIA in Example 7 using monoclonal antibodies-polyclonal antibodies (monopoly method). The amount of hemoglobin contained in the hemolysis solution was also determined by cyanmethohemoglobin method (hemoglobin-testwako, Wako Junyaku). The results are shown in Table 9.

**Table 9**

| No. of specimen | A: Aldose reductase (ng/ml hemolysate) | | B: Hemoglobin (mg/ml hemolysate) | A/B | |
|---|---|---|---|---|---|
| | Poly-poly method | Mono-poly method | | | |
| 25 | 907 | 940 | 188 | 4.8 | 5.0 |
| 54 | 794 | 789 | 184 | 4.3 | 4.3 |
| 102 | 1109 | 1213 | 220 | 5.0 | 5.5 |
| 103 | 1295 | 1292 | 281 | 4.6 | 4.6 |
| 104 | 977 | 1117 | 236 | 4.1 | 4.7 |
| 135 | 1250 | 1175 | 228 | 5.5 | 5.2 |
| 136 | 1597 | 1555 | 227 | 7.0 | 6.9 |
| 155 | 1629 | 1642 | 207 | 7.9 | 7.9 |
| 160 | 919 | 1034 | 202 | 4.5 | 5.1 |
| 161 | 920 | 883 | 212 | 4.3 | 4.2 |

- Poly-poly method:: Polyclonal antibodies-polyclonal antibodies
- Mono-poly method:: Monoclonal antibodies-polyclonal antibodies

Determination of the amount of aldose reductase was possible by either poly-poly method and mono-poly method.

## Claims

1. A method of immunoassay for human aldose reductase by the use of a monoclonal antibody capable of binding to human aldose reductase and/or a polyclonal antibody capable of binding to human aldose reductase.

2. The method of immunoassay for human aldose reductase according to Claim 1 wherein a sandwich method is used.

3. A monoclonal antibody capable of binding to human aldose reductase.

4. A hybridoma producing the monoclonal antibody according to Claim 3.
